# EUROPEAN PATENT APPLICATION

(11) **EP 1 800 642 A1**
(43) Date of publication of application: **27.06.2007**
(21) Application number: 05819980.3
(22) Date of filing: 22.12.2005
(51) Int. Cl.: A61H 13/00, A61C 17/00, A61B 13/00

(54) **GUM MASSAGING MACHINE**

(30) Priority: 22.12.2004 JP 2004371473
(71) Applicant: MATSUSHITA ELECTRIC WORKS, LTD., Kadoma-shi, Osaka-fu 571-8686 (JP)
(72) Inventor: KUNITA, Tomohiro, MATSUSHITA ELECTRIC WORKS LTD, Kadoma-shi, Osaka 5718686 (JP); TANIGUCHI, Shinichi, MATSUSHITA ELECTRIC WORKS LTD, Kadoma-shi, Osaka 5718686 (JP); MOTOHASHI, Ryo, MATSUSHITA ELECTRIC WORKS LTD, Kadoma-shi, Osaka 5718686 (JP); KISHIMOTO, Takashi, MATSUSHITA ELECTRIC WORKS LTD, Kadoma-shi, Osaka 5718686 (JP); HANATO, Yumi, MATSUSHITA ELECTRIC WORKS LTD, Kadoma-shi, Osaka 5718686 (JP)
(74) Representative: DTS Zürich
(86) International application number: PCT/JP2005/023581
(87) International publication number: WO 2006/068215

(57) **Abstract**

A gum massager is made to activate cells in a gingiva efficiently, and thereby, effective to prevent a periodontal disease. The gum massager is comprised of a main body 1 and an attachment 2. The main body 1 has a linear drive motor 5 and a drive shaft 7 which is driven in linear reciprocation vibration in a frequency in sonic wave range, for example, about 300 Hz to 400 Hz. The attachment 2 has a brush unit 3 disposed at a front portion and a heat unit 8 disposed in an inside of the brush unit 3, and attached to a front end of the drive shaft 7. The attachment 2 is vibrated following to the vibration of the drive shaft 7 while a temperature of the brush unit 3 is kept in a region from 40 degrees Celsius to 50 degrees Celsius by the heat unit 8. By applying stimulation due to the vibration in the sonic wave region and the thermal stimulation to the gingiva, the cells of the gingiva are activated.

## Description

### Technical Field

The present invention relates to a gum massager to massage the gum.

### Background Art

Conventionally, there is a gum massager to massage the gum which applies thermal stimulation to the gum as, for example, shown in Japanese Laid-Open Patent Publication No. 50-91194. In addition, for example, Japanese Registered Utility Model No. 1036482 discloses the gum massager that applies mechanical stimulation to the gum. Such gum massagers are intended to prevent a periodontal disease with stimulating cells of a gingiva.

However, it is desired to develop such a gum massager which enables to activate the cells of the gingival more efficiently, and to prevent the periodontal disease, effectively.

### Disclosure of Invention

The present invention was conceived in view of the problems as mentioned above, and aimed to provide a gum massager which enables to activate the cells of the gingiva more efficiently, and to prevent the periodontal disease, effectively.

A gum massager in accordance with an aspect of the present invention comprises a brush unit which is to be contacted with a gum, an actuator to vibrate the brush unit slightly in a frequency equal to or larger than 200 Hz, and a heat source that keeps a temperature of the brush unit in a region from 40 degrees Celsius to 50 degrees Celsius.

In the above mentioned configuration, it is preferable that the actuator vibrates the brush unit slightly in a frequency from 300 Hz to 400 Hz.

Furthermore, it is preferable that the heat source is disposed in an inside of the brush unit.

Still furthermore, it is preferable that the brush unit is formed of a silicon rubber or an elastic material which consists primary of a silicon rubber.

In the gum massager of the present invention, since the brush unit vibrates in the frequency equal to or larger than 200 Hz and it is kept in 40 degrees Celsius to 50 degrees Celsius by the heat source, both of vibration stimulation of the frequency equal to or larger than 200 Hz and thermo stimulation are applied to the gum, and thereby, the cells of the gingiva are activated more efficiently.

### Brief Description of Drawings

FIG. 1A is a front view of a gum massager in accordance with an embodiment of the present invention, and FIG. 1B is side view of the gum massager.
FIG. 2 is a chart explaining a portion of cell coefficients in an experiment in which the above gum massager was used.
FIG. 3A is a chart explaining measurement portions in above experiment, and FIG. 3B is a drawing explaining measurement portions in the above experiment.
FIG. 4 is a graph showing results of measurement of CEJ-AB in the above experiment.
FIG. 5 is a graph showing results of measurement of wFG in the above experiment.
FIG. 6 is a graph showing results of measurement of hFG in the above experiment.
FIG. 7 is a graph showing results of measurement of CEJ-aICT/hFG in the above experiment
FIG. 8 is a graph showing results of measurement of numbers of vascular cavities in conjugation subepithelial connective tissue in junction in the above experiment.
FIG. 9 is a graph showing results of measurement of wOE in the above experiment.
FIG. 10 is a graph showing results of measurement of wIE in the above experiment.

### Best Mode for Carrying Out the Invention

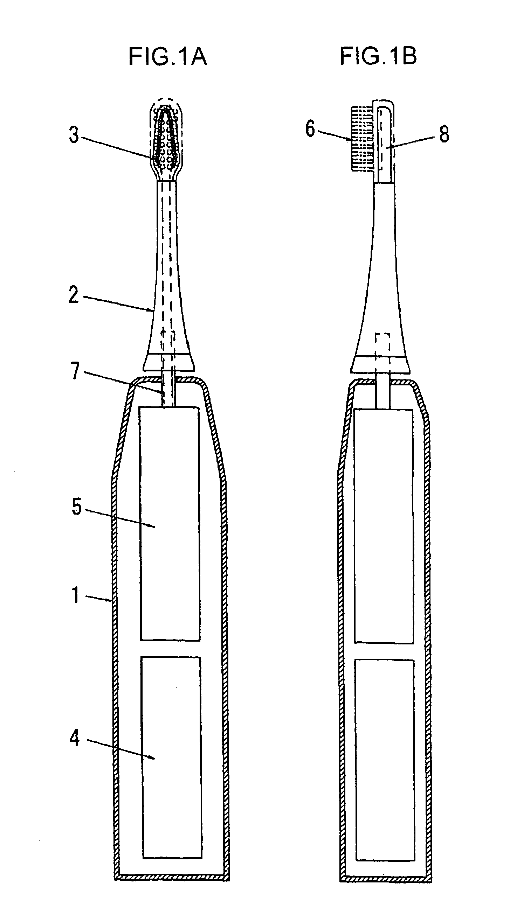

A gum massager in accordance with an embodiment of the present invention is described in detail with reference to drawing. FIG. 1A and FIG. 1B show the gum massager in accordance with this embodiment. The gum massager is comprised of a main body 1 and an attachment 2 which is detachably attached to a drive shaft 7 projecting from an end of the main body 1. In addition, the attachment 2 shown in the figures is a thing used for massaging gum as mentioned later. The gum massager is usable as an electric toothbrush when the attachment 2 is interchanged for an attachment (it is not illustrated) having a brush for brushing tooth.

A battery 4 as power source and a linear vibrating motor (actuator) 5 which can give linear reciprocating vibration to the drive shaft 7 in an axial direction thereof are disposed in an inside of the main body 1. This linear vibrating motor 5 drives the drive shaft 7 in linear reciprocation vibration in a frequency in sonic wave range, for example, about 300 Hz to 400 Hz.

The attachment 2 has a brush unit 3 allocated at a front end, and a heat unit (a heat source) 8 allocated in an inside of the brush unit 3. The attachment 2 is attached to a top end of the drive shaft 7 of the main body 1 so as to be vibrated following to the linear reciprocating vibration of the drive shaft 7. A brush 6, which is made of, for example, a silicon rubber, and serves as a portion to be contacted to the gum, is provided on a side face of the brush unit 3 to be protruded. The heat unit 8 is, for example, a metal wire, and configured to heat when an electric power is supplied from the battery 4. In this embodiment, the heat unit 8 is configured to heat to keep the temperature of the brush unit 3 in a range from about 40 degrees Celsius to 50 degrees Celsius.

Subsequently, an experiment and results of it when advantageous effect of the above gum massage was verified in comparison with the case of using a toothbrush operated by hand is described. In this experiment, the brush unit 3 was previously dipped into a thermostatic chamber at 50 degrees Celsius so as to keep the temperature of the brush unit 3 in a region from about 40 degrees Celsius to 50 degrees Celsius while the experiment, instead of using the heat unit 8, as described later.

This experiment was carried out for gingivas of dogs. The test subjects were beagles (eight male dogs), and the second and third premolar teeth of the upper jaw and the third and fourth premolar teeth of the lower jaw were selected as the subject teeth. Prior to the first experiment, after removing dental calculi on upper border of gingiva and lower border of gingiva, removing plaques of the subject by curet were performed once a day for a fortnight. The experiment period was selected to be five weeks. During experiment period, soft food including water was fed to the subject dogs as a diet. Then, gum massage by blushing of five seconds was carried out for one subject tooth once a day under general anesthesia for these subject dogs.

For gum massage, four kinds of toothbrush and gum massagers of (a) a toothbrush for hand operation, (b) a thing where an attachment 2 having a brush unit 3 allocated a brush 6 made of a nylon was attached to the main body 1, (c) a thing where an attachment 2 having a brush unit 3 allocated a brush 6 made of a silicon rubber was attached to the main body 1, and (d) a thing where the attachment 2 having the brush unit 3 of the above (c) which was previously dipped into the thermostatic chamber at 50 degrees Celsius more than ten minutes was attached to the main body 1 were used. Gum massage was carried out in a manner so that an oral cavity of each dog was divided into four areas of upper right, upper left, lower right and lower left, and the above mentioned four kings of the toothbrush and the gum massagers were allocated to the subject teeth in the four areas.

After termination of the experiment period, the subject dogs were butchered by administering excessive quantity of anesthesia liquid into veins, and the subject teeth and gingivas were taken out. Then, the subject teeth and gingivas were processed of fixing (4% paraformaldehyde), decalcification ((10% EDTA solution) and paraffin treatment, and after that, immunostaining with using monoclonal antibody or hematoxylin eosin staining for PCNA (Proliferating Cell Nuclear Antigen) was performed to them.

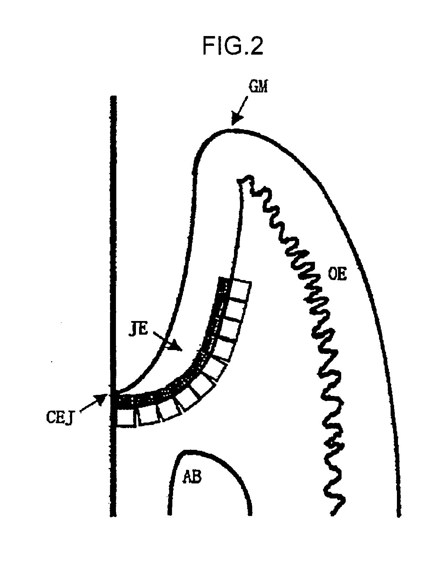

From the stained specimens of PCNA obtained by the above mentioned processes, a number of PCNA positive reacted conjugation epithelial basement cells and a total number of conjunction epithelial basement cells per unit length, and a number of PCNA positive reacted fibrocytes ("A" in FIG. 2) and a total number of the fibrocytes ("B" in FIG. 2) per unit area in a subepithelial connective tissue were counted so that variation of respective cells were verified, as shown in FIG. 2.

As for the variation of the conjugation epithelial basement cells, in the specimen brushed by the toothbrush for hand operation (a), the number of the PCNA positive reacted conjugation epithelial basement cells (per 0.1 mm) was 2.6 ± 0.6 and the total number of conjunction epithelial basement cells (per 0.1 mm) was 10.1 ± 0.6. In the specimen brushed by the thing (b) using the nylon brush, the number of the PCNA positive reacted conjugation epithelial basement cells was 3.5 ± 1.4, and the total number of conjunction epithelial basement cells was 10.9 ± 1.0. In the specimen brushed by the thing (c) using the silicon brush, the number of the PCNA positive reacted conjugation epithelial basement cells was 3.6 ± 1.4, and the total number of conjunction epithelial basement cells was 10.3 ± 0.4. In the specimen brushed by the thing (d) using the silicon brush dipped in the thermostatic chamber at 50 degrees Celsius, the number of the PCNA positive reacted conjugation epithelial basement cells was 3.9 ± 0.9, and the total number of conjunction epithelial basement cells was 10.9 ± 0.8.

From these results, it is found that turn-over of gingiva conjugation epithelium was accelerated larger when using the gum massager (b, c, d) which vibrates the brush unit 3 in the sonic wave region than when using the toothbrush for hand operation. Furthermore, it is suggested that the turn-over of the gingiva conjugation epithelium was accelerated larger by the thing using the silicon brush (c, d) than the thing using the nylon brush (b). Still furthermore, it is suggested that the turn-over of the gingiva conjugation epithelium was accelerated larger by the thing adding thermal stimulation by dipping the thermostatic chamber at 50 degrees Celsius (d) that the thing using the silicon brush with no thermal stimulation (c). When the turn-over of the gingiva conjugation epithelium was accelerated, it is possible to prevent the infection of the disease-causing germ adhered on the epithelium, and thereby, to prevent the periodontal disease.

In addition, as for the variation of the fibrocytes in the subepithelial connective tissue, in the specimen brushed by the toothbrush for hand operation (a), the number of PCNA positive reacted fibrocytes (per 0.1 mm) was 2.8 ± 1.0, and the total number of the fibrocytes was 14.2± 1.8. In the specimen brushed by the thing (b) using the nylon brush, the number of PCNA positive reacted fibrocytes was 5.1 ± 1.5, and the total number of the fibrocytes was 17.1± 2.2. In the specimen brushed by the thing (c) using the silicon brush, the number of PCNA positive reacted fibrocytes was 4.5 ± 1.0, and the total number of the fibrocytes was 16.9± 2.0. In the specimen brushed by the thing (d) using the silicon brush dipped in the thermostatic chamber at 50 degrees Celsius, the number of PCNA positive reacted fibrocytes was 6.5 ± 1.4, and the total number of the fibrocytes was 19.0± 1.4.

From these results, it is found that the number of PCNA positive reacted fibrocytes and the total number of the fibrocytes were significantly increased larger when applying vibrations of the sonic wave region (b, c, d) than when using the toothbrush for hand operation. Furthermore, since the number of PCNA positive reacted fibrocytes was significantly increased larger when adding heat (d) than when adding no heat (c), it is suggested that the increase of the number of cells of fibrocytes is accelerated by adding the thermal stimulation. Because the fibrocytes are cells that generate collagen which is main composition of a gingiva, the increase of these cells is effective to restore the destroyed gingiva conformation by inflammation.

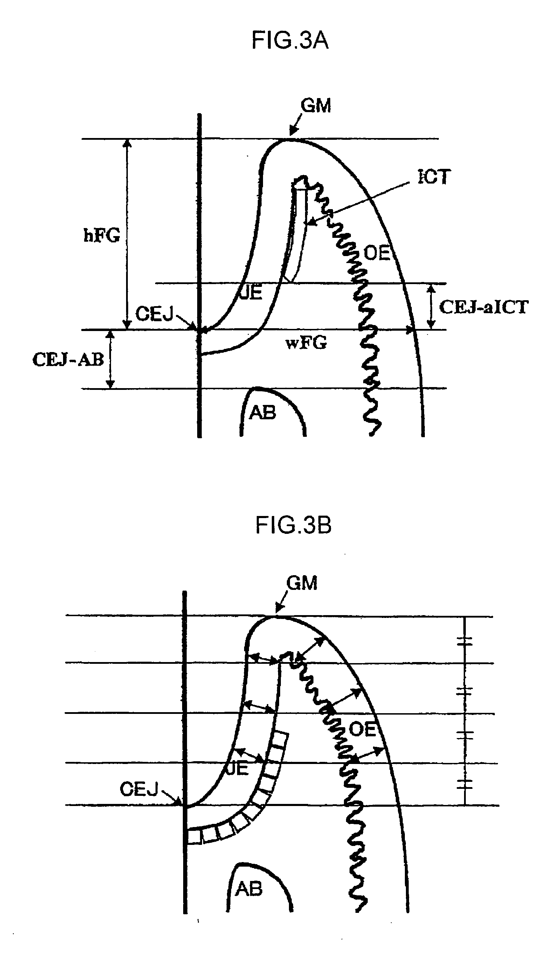

In addition, from the stained specimens of hematoxylin eosin obtained by the above mentioned processes, a distance CEJ-AB (a distance from a cement enamel boundary (CEJ) to a peak of an alveolar bone), a width wFG (a width of an isolation gingiva from the origin of the CEJ), a height hFG (a height of the isolation gingiva from the origin of the CEJ),a distance CDJ-aICT/hFG (a proportion of a distance from the CEJ to the most apex of root of inflammatory cell moist accounting in the hFG), a number of the vascular cavities per unit area of the conjugation subepithelial connective tissue, a width wOE (a width of an outer edge of the epithelium), and a width wIE (a width of an inner edge of the epithelium) were respectively measured, as shown in FIG. 3A and FIG. 3B.

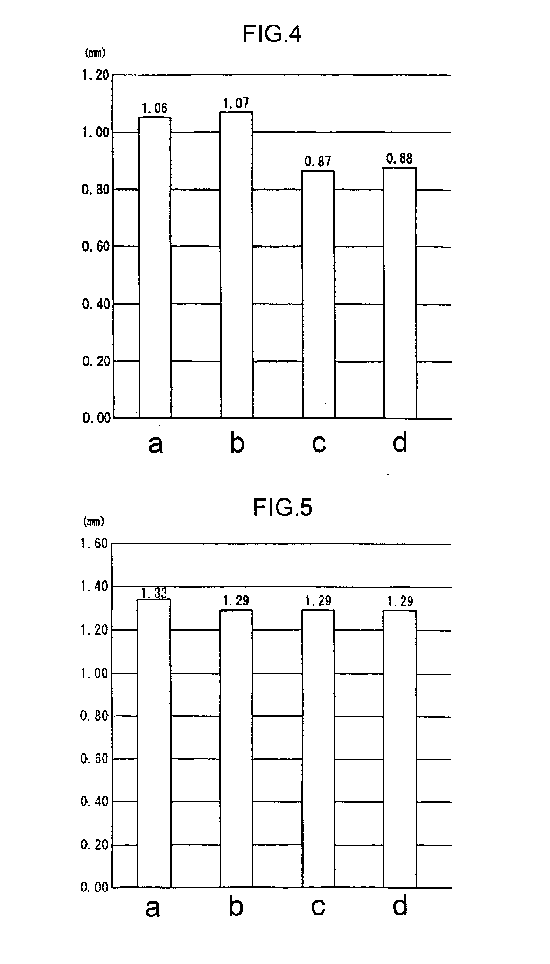

FIG. 4, FIG. 5, and FIG. 6 respectively show the measurement results of the CEJ-AB, the wFG, and the hFG. The CEJ-AB, the wFG and the hFG show circumstances of absorption of the alveolar bone. With respect to the CEJ-AB, the wFG and the hFG, there was no significant difference among the above mentioned four groups (a, b, c and d). Therefore, it is found that the stimulation due to vibration in the sonic wave region unlikely causes harm to the gingiva conformation than the stimulation due to the toothbrush for hand operation.

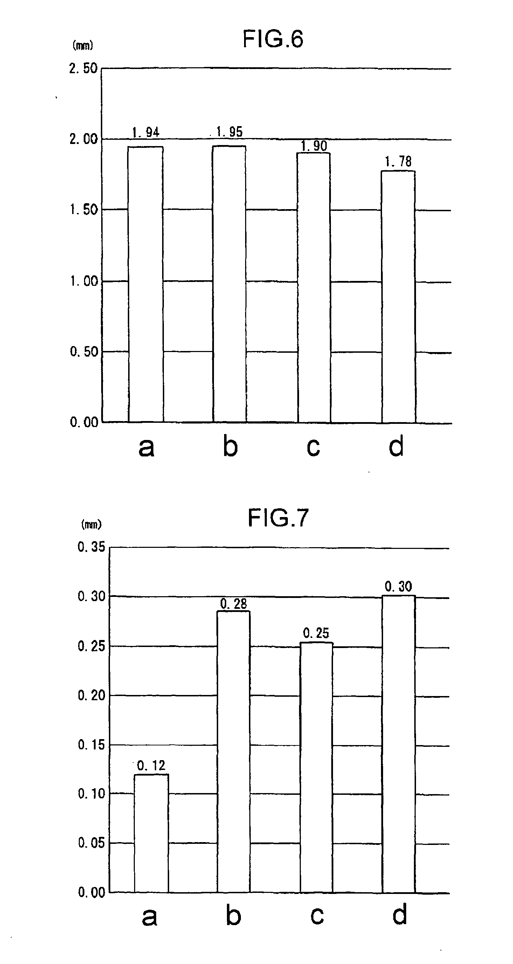

In addition, FIG. 7 shows the measurement result of the CDJ-aICT/hFG. As can be seen from this measurement result, since the degree of the inflammatory cell moist was significantly decreased when applying the stimulation due to the vibration in the sonic wave region (b, c, d) than when applying the stimulation due to the toothbrush for hand operation, it is found that the application of the stimulation due to the vibration in the sonic wave region is effective to improve inflammation of the gingiva.

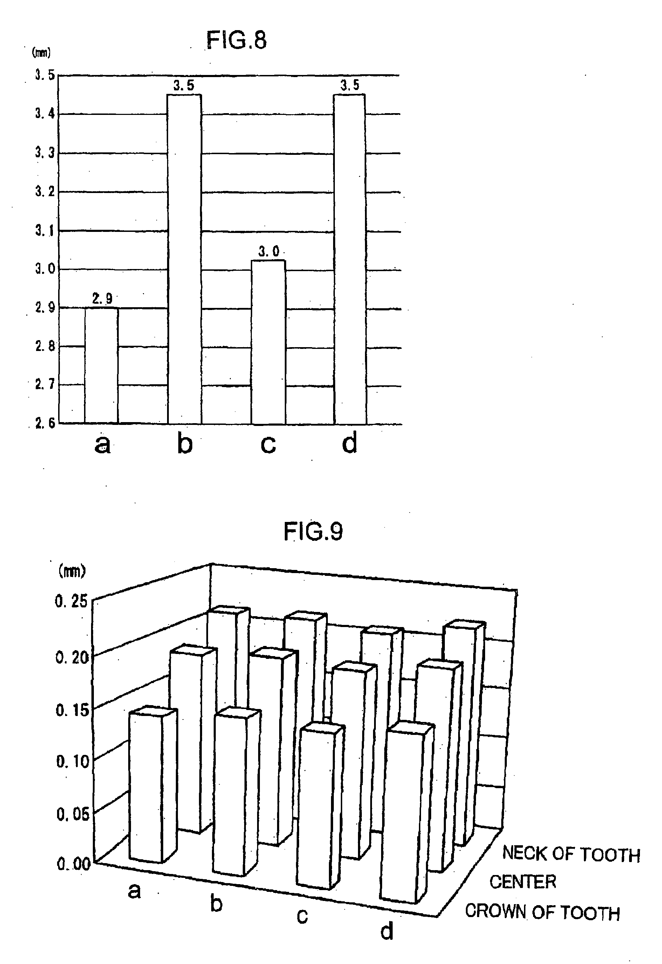

FIG. 8 shows the number of the vascular cavities per 0.01 square millimeter of the conjugation subepithelial connective tissue. As shown in the figure, it is found that the number of the vascular cavities was significantly increased when applying the stimulation due to the vibration in the sonic wave region (b, c, d) than when applying the stimulation due to the toothbrush for hand operation. Therefore, it is possible to judge that the stimulation due to the vibration in the sonic wave region is effective to activate the cells of the gingiva. In addition, it is suggested that new blood vessel growth is spurred with applying the stimulation due to the vibration in the sonic wave region and the thermal stimulation together.

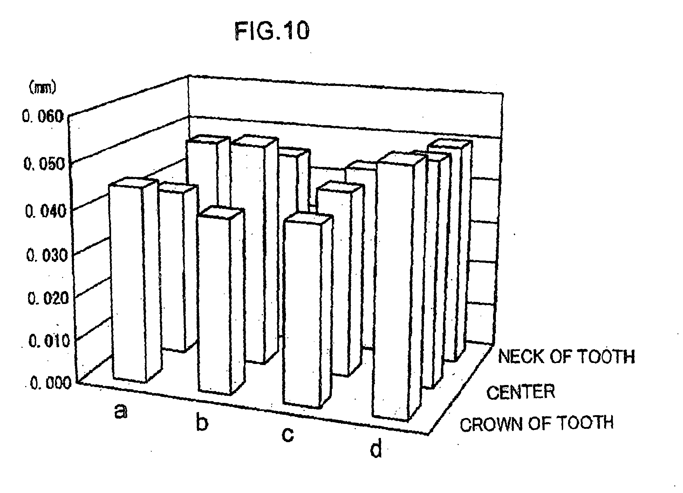

Furthermore, FIG. 9 and FIG. 10 respectively show the measurement results of the wOE and the wIE. As shown in FIG. 9, with respect to the epithelium on the outer edge of the gingiva, there was no significant difference among the above mentioned four groups. On the other hand, as shown in FIG. 10, it is found that the width of the epithelium on the inner edge of the gingiva was made thicker in the center area when applying the stimulation due to the vibration in the sonic wave region (b, c, d) than when applying the stimulation due to the toothbrush for hand operation. In addition, it is found that the width of the epithelium on the inner edge of the gingiva was made thicker toward the crown of tooth when applying the stimulation due to the vibration in the sonic wave region and the thermal stimulation through the silicon brush (d) than when applying no thermal stimulation together (c). Therefore, it is possible to judge that the stimulation due to the vibration in the sonic wave region is effective to activate the cells of the gingiva, and it is suggested that new blood vessel growth is spurred with applying the thermal stimulation together.

As mentioned above, it is effective to apply the stimulation due to the vibration in the sonic wave region to the gingiva in order to activate the cells of the gingiva while carrying out the gum massage. In addition, by applying the thermal stimulation further to the stimulation due to the vibration in the sonic wave region, the cells of the gingiva can be activated efficiently.

Besides, the present invention is not limited to the configuration of the above mentioned embodiments, and appropriately various kinds of transformation are applicable in a scope that does not extend beyond the invention. For example, the linear drive motor 5 should be configured to vibrate the drive shaft 7 in a frequency of sonic wave region equal to or larger than 200 Hz. At this time, the cells of the gingiva can be activated efficiently by applying the stimulation due to the vibration in the sonic wave region and the thermal stimulation. In addition, the brush 6 of the brush unit 3 may be formed of an elastic material which consists primary of the silicon rubber, instead of the silicon rubber.

This application is based on Japanese patent application 2004-371473 filed in Japan, the contents of which are hereby incorporated by references.

Although the present invention has been fully described by way of example with reference to the accompanying drawings, it is to be understood that various changes and modifications will be apparent to those skilled in the art. Therefore, unless otherwise such changes and modifications depart from the scope of the present invention, they should be construed as being included therein.

## Claims

1. A gum massager comprising:
a brush unit which is to be contacted with a gum;
an actuator to vibrate the brush unit slightly in a frequency equal to or larger than 200 Hz; and
a heat source that keeps a temperature of the brush unit in a region from 40 degrees Celsius to 50 degrees Celsius.

2. The gum massager in accordance with claim 1, **characterized in that** the actuator vibrates the brush unit slightly in a frequency from 300 Hz to 400 Hz.

3. The gum massager in accordance with claim 1 or 2, **characterized in that** the heat source is disposed in an inside of the brush unit.

4. The gum massager in accordance with one of claims 1 to 3, **characterized in that** the brush unit is formed of a silicon rubber or an elastic material which consists primary of a silicon rubber.
